# EUROPÄISCHE PATENTSCHRIFT

(11) **EP 2 661 308 B1**
(45) Veröffentlichungstag und Bekanntmachung des Hinweises auf die Patenterteilung: **08.05.2019**
(21) Anmeldenummer: 12701434.8
(22) Anmeldetag: 04.01.2012
(51) Int. Cl.: A61K 31/6615, A61K 31/688, A61K 33/14, A61K 38/17, A61P 43/00

(54) **HYPOTONE WÄSSRIGE ZUSAMMENSETZUNG MIT VERRINGERTEM CHLORIDGEHALT MIT ODER OHNE PHOSPHOLIPIDE**
HYPOTONIC AQUEOUS COMPOSITION WITH REDUCED CHLORIDE CONTENT, WITH OR WITHOUT PHOSPHOLIPIDS
COMPOSITION AQUEUSE HYPOTONIQUE POSSÉDANT UNE TENEUR RÉDUITE EN CHLORURE AVEC OU SANS PHOSPHOLIPIDES

(30) Priorität: 05.01.2011 EP 11000044
(43) Veröffentlichungstag der Anmeldung: 13.11.2013
(73) Patentinhaber: Prenatal International GmbH, 06126 Halle (Saale) (DE)
(72) Erfinder: TCHIRIKOV, Michael, 06126 Halle (Saale) (DE)
(74) Vertreter: Mai Dörr Besier European Patent Attorneys
(86) Internationale Anmeldenummer: PCT/EP2012/000029
(87) Internationale Veröffentlichungsnummer: WO 2012/093087

(56) Entgegenhaltungen:
- EP-A1- 0 998 916
- EP-A1- 1 719 517
- BRACE R A ET AL: "Amniotic fluid volume responses to amnio-infusion of amniotic fluid versus lactated Ringer's solution in fetal sheep", JOURNAL OF THE SOCIETY FOR GYNECOLOGIC INVESTIGATION, ELSEVIER, NEW YORK, NY, US, Bd. 11, Nr. 6, 1. September 2004 (2004-09-01), Seiten 363-368, XP004550634, ISSN: 1071-5576, DOI: DOI:10.1016/J.JSGI.2004.02.006 in der Anmeldung erwähnt
- CORPENING JAMES W ET AL: "Ingested bovine amniotic fluid enhances morphine antinociception in rats", PHYSIOLOGY AND BEHAVIOR, Bd. 70, Nr. 1-2, Juli 2000 (2000-07), Seiten 15-18, XP002638933, ISSN: 0031-9384
- DOI SHIGEHARU ET AL: "Effect of maternal hydration on oligohydramnios: A comparison of three volume expansion methods", OBSTETRICS AND GYNECOLOGY, Bd. 92, Nr. 4 PART 1, Oktober 1998 (1998-10), Seiten 525-529, XP002638934, ISSN: 0029-7844
- TCHIRIKOV MICHAEL ET AL: "Long-term amnioinfusion through a subcutaneously implanted amniotic fluid replacement port system for treatment of PPROM in humans.", EUROPEAN JOURNAL OF OBSTETRICS, GYNECOLOGY, AND REPRODUCTIVE BIOLOGY SEP 2010 LNKD- PUBMED:20488612, Bd. 152, Nr. 1, September 2010 (2010-09), Seiten 30-33, XP002638935, ISSN: 1872-7654 in der Anmeldung erwähnt
- unknown: "Ringerlösung", Wikipedia , 12. Mai 2008 (2008-05-12), XP002672067, Gefunden im Internet: URL:http://de.wikipedia.org/wiki/Ringerl%C 3%B6sung [gefunden am 2012-03-22]

## Beschreibung

Die vorliegende Erfindung betrifft eine hypotone wässrige Zusammensetzung mit verringertem Chloridgehalt mit oder ohne Phospholipide zur Anwendung in einem Verfahren zur chirurgischen oder therapeutischen Behandlung des menschlichen oder tierischen Körpers oder in einem Diagnostizierverfahren, das am menschlichen oder tierischen Körper vorgenommen wird. Besonders im Vordergrund steht dabei die Verwendung der wässrigen Zusammensetzung zur Anwendung in einem Verfahren zur therapeutischen Behandlung des menschlichen Körpers, vor allem als Fruchtwasserersatz (Amniotic Fluid), insbesondere bei der Amnioninfusion (Fruchtwasserauffüllung), bevorzugt bei einem vorzeitigen Blasensprung.

Ein vorzeitiger Blasensprung (Preterm Premature Rupture Of Membranes (PPROM)) ist einer der Hauptgründe perinataler Morbidität und Mortalität. Er tritt bei 3% aller Schwangerschaften auf und ist für ein Drittel aller Frühgeburten verantwortlich (Mercer BM. Preterm premature rupture of the membranes. Obstet Gynecol 2003; 101: 178-193). Wichtige Risikofaktoren für PPROM sind Infektionen (Goldenberg RL, Culhane JF, Iams JD, Romero R. Epidemiology and causes of preterm birth. Lancet 2008; 371: 75-84), Polyhydramnion (Simhan HN, Canavan TP. Preterm premature rupture of membranes: diagnosis, evaluation and management strategies. BJOG 2005; 112: 32-37), invasive Pränataldiagnostik (Van Kamp IL, Klumper FJ, Oepkes D, et al. Complications of intrauterine intravascular transfusion for fetal anemia due to material red-cell alloimmunization. Am J Obstet Gynecol 2005: 192:171-177) und fetoskopische Operationen (Gratacos E, Deprest J. Current experience with fetoscopy and the Eurofoetus Registry for fetoscopic procedures. Eur J Obstet Gynecol Reprod Biol 2000; 92: 151-159). Bei Auftreten einer pulmonalen Hypoplasie beträgt das perinatale Mortalitätsrisiko ca. 80%.

Therapeutische Maßnahmen zur Behandlung von PPROM haben die Wiederherstellung und die Aufrechterhaltung des normalen Flüssigkeitsvolumens in der Amnionhöhle zum Ziel. Zur Erhöhung des amniotischen Flüssigkeitsindex (AFI) wird das Flüssigkeitsvolumen in der Amnionhöhle über eine Amnioninfusion kontinuierlich erhöht (Tan LK et al. Test amnioinfusion to determine suitability for serail therapeutic amnioinfusion in midtrimester premature rupture of membranes. Fetal Diagn Ther 2003; 18: 183-189; Tranquillli AL et al. Transabdominal amnioinfusion in preterm premature rupture of membranes: a randomised controlled trial. BJOG 2005; 112: 759-763; Hsu TL et al. The experience of amnioinfusion for oligohydramnios during the early second trimester. Taiwan J Obstet Gynecol 2007; 46 (4)).

Zur Auffüllung des Flüssigkeitsvolumens in der Amnionhöhle wird üblicherweise eine isotonische Salzlösung verwendet, um eine Schädigung des Fötus nach Möglichkeit zu vermeiden. Getestet wurden als isotonische Salzlösung u. a. eine physiologische Kochsalzlösung (0,9 Gew.-% NaCl; Osmolarität: 308 mosm/l), eine Ringerlösung, eine Ringer-Laktat-Lösung und eine Ringer-Acetat-Lösung.

Die Ringer-Infusionslösung enthält:
8,60 g/l (147 mmol/l) NaCl
0,30 g/l (4,0 mmol/l) KCl und
0,33 g/l (2,2 mmol/l) CaCl₂.

Die Ringer-Laktat-Lösung enthält:
5,9 g/l - 6,1 g/l (125 mmol/l - 134 mmol/l) NaCl
0,3 g/l -0,4 g/l (4,0 mmol/l - 5,4 mmol/l) KCl
0,22 g/l -0,29 g/l (0,9 mmol/l - 2,0 mmol/l) CaCl₂*2H₂O und
2,8 g/l -3,45 g/l (25 mmol/l - 31 mmol/l) CH₃CHOHCOONa.
Ihre theoretische Osmolarität liegt zwischen 262 mosm/l und 293 mosm/l.

Die Ringer-Acetat-Lösung enthält:
6,00 g/l (130 mmol/l) NaCl
0,40 g/l (5,4 mmol/l) KCl
0,134 g/l (0,9 mmol/l) CaCl₂*2H₂O
0,203 g/l (1,0 mmol/l) MgCl₂*6H₂O
3,70 g/l (27 mmol/l) CH₃COONa*3H₂O
Ihre theoretische Osmolarität liegt bei 276 mosm/l.

Dabei hat sich die physiologische Kochsalzlösung am meisten bewährt.

Die bislang bekannten Verfahren zur Amnioninfusion zur Behandlung von PPROM können allerdings nicht befriedigen, da die von außen eingefüllte Flüssigkeit (isotonische Salzlösung) sehr schnell wieder aus der Gebärmutter abfließt, wodurch die Wirkung der Amnioninfusion stark vermindert ist. Die bekannten Verfahren betrafen beispielsweise eine zervikale Okklusion mit einem Fibringel (Zamlynski J, Bodzek P, Olejek A, Grettka K, Manka G. Results of amnioinfusion in pregnancies with oligohydramnios and non-ruptured fetal membranes. Med Wieku Rozwoj 2003; 7:187-194) oder die Infusion einer Flüssigkeit über einen transzervikalen Katheter (Machalski T, Sikora J, Bakon I, Magnucki J, Grzesiak-Kubica E, Szkodny E. Short-term and long-term fetal heart rate variability after amnioinfusion treatment of oligohydramnios complicated pregnancy. Ginekol Pol 2001; 72:1107-1111).

Auch wiederholte, transabdominale Amnioninfusionen mit physiologischer Kochsalzlösung zur Behandlung von PPROM zeigten bei einer Behandlung innerhalb von 6h nach dem Flüssigkeitsverlust nur minimale Vorteile (De Santis M, Scavo M, Noia G, Masini L, Piersigilli F, Romagnoli C, Caruso A. Transabdominal amnioinfusion treatment of severe oligohydramnios in preterm premature rupture of membranes at less than 26 gestational weeks. Fetal Diagn Ther 2003; 18:412-417; Ogunyemi D, Thompson W. A case controlled study of serial transabdominal amnioinfusions in the management of second trimester oligohydramnios due to premature rupture of membranes. Eur J Obstet Gynecol Reprod Biol. 2002; 102:167-172).

Vor diesem Hintergrund wurde eine Langzeit-Amnioninfusion von physiologischer Kochsalzlösung mittels eines subkutan implantierten Fruchtwasserersatzportsystems zur Behandlung von PPROM beim Menschen vorgeschlagen. Diese Vorgehensweise ermöglichte eine Verlängerung der Schwangerschaft um mehrere Wochen unter Vermeidung einer Lungenhypoplasie (Tchirikov M, Steetskamp J, Hohmann M, Koelbl H. Long-term amnioinfusion through a subcutaneously implanted amniotic fluid replacement port system for treatment of PPROM in humans. Eur J Obstet Gynecol Reprod Biol. 2010; 152:30-33).

Auch Untersuchungen über die Zusammensetzung des Fruchtwassers wurden bereits publiziert. Man geht davon aus, dass die im menschlichen Fruchtwasser enthaltenen Phospholipide für die Lungenreifung des Fötus verantwortlich sind (Lohninger A, Salzer H, Simbruner G, Husslein P, Martin G. Relationship among human amniotic fluid dipalmitoyl lecithin, postpartum respiratory compliance and neonatal respiratory distress syndrome. Clin. Chem. 1983; 29: 650-655; Almog R, Anderson-Samsonoff C, Berns DS, Saulsbery R. A methodology for determination of phospholipids. Analytical Biochemistry 1990; 188:237-242). Entsprechende Präparate auf der Basis von Phospholipiden aus der Rinderlunge sind für die Behandlung von Frühgeborenen mit Atemnot-Syndrom seit Längerem auf dem Markt (ALVEOFACT®, SURVANTA®).

ALVEOFACT® enthält, bezogen auf 1,2 ml, 50,76 mg - 60 mg Phospholipidfraktion aus Rinderlunge (Surfactant; entspricht 50 mg Gesamtphospholipide) sowie Natriumchlorid und Natriumhydrogencarbonat (Rote Liste 2000).

SURVANTA® enthält, bezogen auf 8 ml, 72,8 mg - 211,2 mg Phospholipidfraktion aus Rinderlunge, 18,4 mg - 157,6 mg 1,2-Dipalmitoyl-sn-glycero(3)phosphocholin, 1,4 mg - 11,1 mg Palmitinsäure, 2,2 mg - 10,5 mg Glyceroltripalmitat (entspricht 50 mg Gesamtphospholipide) sowie Natriumchlorid und Natriumhydrogencarbonat oder Salzsäure (Rote Liste 2000).

Weiterhin wurde festgestellt, dass die Konzentration der Surfactant-Proteine sich bei intraamniotischen Infektionen ändert (Chaiworapongsa T, Hong JS, Hull WM, Romero R, Whitsett JA. Amniotic fluid concentration of surfactant proteins in intraamniotic infection. J Matern Fetal Neonatal Med. 2008; 21: 663-670) und bei einer Spontangeburt am erwarteten Geburtstermin abfällt (Chaiworapongsa T, Hong JS, Hull WM, Kim CJ, Gomez R, Mazor M, Romero R, Whitsett JA. The concentration of surfactant protein-A in amniotic fluid decreases in spontaneous human parturition at term. J Matern Fetal Neonatal Med. 2008; 21: 652-659).

Darüber hinaus haben eigene Untersuchungen ergeben, dass die Osmolarität, d. h. die Anzahl der osmotisch aktiven Teilchen pro Liter Lösung, im menschlichen Fruchtwasser niedriger als in physiologischer Kochsalzlösung (308 mosm/l) ist. Weiterhin ist auch die Chloridionenkonzentration im Fruchtwasser deutlich geringer als in physiologischer Kochsalzlösung.

Vor diesem Hintergrund ist davon auszugehen, dass eine Amnioninfusion, insbesondere eine Langzeit-Amnioninfusion, mit einer physiologischen Kochsalzlösung, wie sie derzeit nach dem Stand der Technik favorisiert wird, für einen Fötus aus mehreren Gründen nachteilig ist:
Zum einem wird durch die Verabreichung einer Infusionslösung mit einer zu hohen Osmolarität, die Osmolarität des Fruchtwassers erhöht, welches von dem Fötus ständig getrunken und wieder ausgeschieden wird. Dies kann, insbesondere bei Langzeit-Verabreichung der Infusionslösung, eine Schädigung der Organe des Fötus, vor allem seiner Haut, seiner Augen und seiner Verdauungsorgane, insbesondere seiner Nieren, bewirken. Weiterhin ist eine Veränderung der fetalen Programmierung zu befürchten, die ggf. zu einer Hypertonie im späteren Leben führen kann, auch wenn dies aufgrund fehlender entsprechender Daten bisher noch nicht beschrieben wurde.

Ähnliches gilt für die Verabreichung einer Infusionslösung mit einer zu hohen Chloridionenkonzentration. Diese erhöht die Chloridionenkonzentration des Fruchtwassers, welches von dem Fötus ständig getrunken und wieder ausgeschieden wird. Dies kann, insbesondere bei Langzeit-Verabreichung der Infusionslösung, eine Schädigung der Organe des Fötus, vor allem seiner Haut, seiner Augen und seiner Verdauungsorgane, insbesondere seiner Nieren, bewirken. Weiterhin ist eine Veränderung der fetalen Programmierung zu befürchten, die ggf. zu Problemen im späteren Leben führen kann, auch wenn dies aufgrund fehlender entsprechender Daten bisher noch nicht beschrieben wurde.

Darüber hinaus wird durch die Verabreichung einer Infusionslösung, die keine Phospholipide enthält, die Konzentration der Phospholipide im Fruchtwasser abgesenkt, welches mit den Lungenbläschen des Fötus im ständigen Kontakt ist. Dies kann, insbesondere bei Langzeit-Verabreichung der Infusionslösung, zu einem Ausspülen der Phospholipide auf den Lungenbläschen des Fötus und somit zu einer Verlangsamung der Lungenreifung des Fötus führen. Die Überlebenschancen eines solchen Fötus werden dadurch deutlich erniedrigt.

Weiterhin wird durch die Verabreichung einer Infusionslösung, die keine Surfactant-Proteine enthält, die Konzentration der Surfactant-Proteine im Fruchtwasser abgesenkt. Dies kann, insbesondere bei Langzeit-Verabreichung der Infusionslösung, zu einer Schädigung der Organe des Fötus führen.

Aus dem Stand der Technik sind auch fetoskopische Operationen bekannt, bei denen das Amnion geöffnet, das Fruchtwasser zumindest teilweise entfernt, der Fötus operiert, das Amnion wieder verschlossen und das entnommene Fruchtwasser zumindest teilweise wieder der Amnionhöhle zugeführt wird.

Aus medizinischen und ethischen Gründen ist eine Verwendung des auf diese Weise entnommenen Fruchtwassers zur Behandlung von PPROM bei anderen Patientinnen nicht möglich. Zum einen wird das Fruchtwasser nach der Operation für den operierten Fötus benötigt, um seine Überlebenschancen zu erhöhen. Darüber hinaus enthält das entnommene Fruchtwasser Gewebereste und beträchtliche Mengen DNA des operierten Fötus (J Cln Endocrinol Metab 2000; 85:214-8 "Large amounts of cell-free fetal DNA are present in amniotic fluid"). Fruchtwasser kann beispielsweise 778 fetale Zellen und mehr in 1 mm³ enthalten. Diese Gewebereste und diese große Menge an DNA können bei einem anderen Fötus oder dessen Mutter zu unerwünschten Reaktionen auf das genetisch-fremde Material und/oder zu Infektionen führen.

Aus ähnlichen Gründen ist auch eine Verwendung von tierischem Fruchtwasser zur Behandlung von PPROM bei Menschen nicht bedenkenlos möglich. Zum einen sind aufgrund der in diesem Fruchtwasser enthaltenen Gewebereste und große Mengen an DNA des tierischen Fötus, unerwünschten Reaktionen des Fötus und/oder der Mutter auf das genetisch-fremde Material und/oder Infektionen zu befürchten. Weiterhin besteht ggf. die Gefahr einer Bildung von Chimären durch Mischung der genetischen Informationen, die in den meisten Ländern von Rechts wegen untersagt ist.

Die Veröffentlichung Brace R A et al. Amniotic fluid volume responses to amnio-infusion of amniotic fluid versus lactated Ringer's solution in fetal sheep. J Soc Gynecol Investig. 2004, 11 (6): 363-368 beschreibt eine Amnio-Infusion von Fruchtwasser bei schwangeren Schafe, wobei das verwendete Fruchtwasser zuvor aus anderen Schafföten gewonnen wurde.

Bei der Verwendung von Fruchtwasser von Schafen bei einer Amnio-Infusion bei einem anderen Säugetier, insbesondere beim Menschen, besteht jedoch ein erhebliches Risiko, dass Erbmaterial des Tieres, z. B. die DNA des Schafes, in die DNA des Föten eingebaut und sogenannte Chimäre gebildet werden. Daher ist die Verwendung von tierischem Fruchtwasser bei einer Amnio-Infusion bei einem anderen Säugetier, insbesondere beim Menschen, nicht sinnvoll, zumal die Herstellung von Chimären in den meisten Ländern strafbar ist.

Darüber hinaus wäre die Verwendung von Fruchtwasser von Schafen für Langzeit-Amnio-Infusionen infolge eines PPROM bei anderen Säugetieren, insbesondere bei Menschen, die im Rahmen der vorliegenden Erfindung im Vordergrund stehen, nicht sinnvoll. So trinkt ein menschlicher Fetus täglich zwischen 200 ml und 700 ml Fruchtwasser und die Haut und auch die Schleimhäute des Fetus sind für das umgebende Fruchtwasser teilweise durchgängig. Bei einem PPROM muss man ca. 2 Liter Fruchtwasser täglich über die Monate infundieren. Sollte man ernsthaft in Erwägung ziehen, diese große Menge Fruchtwasser von Tieren für diese Zwecke zu gewinnen, so besteht die Gefahr, dass in einigen Fällen Infektionen von den Tieren direkt an den Fetus und auch an die Schwangere übertragen werden. Dies ist insbesondere für Infektionen, wie z. B. Prionen-Übertragungen, zu befürchten, die über eine Sterilisierung des tierischen Fruchtwassers nicht vollständig vermieden werden können.

Abgesehen davon sind die Natriumionen-Konzentration (121.4 mmol/L) und die Calciumionenkonzentration (0.75 mmol/L) im Fruchtwasser des Schafes signifikant kleiner als im menschlichen Fruchtwasser (Gesteland K M et al. Intramembranous solute and water fluxes during high intramembranous absorption rates in fetal sheep with and without lung liquid diversion. American Journal of Obstetrics & Gynecology, 2009 85.e1 - 85.e6; Ross M G et al. Amniotic fluid ionic concentration in response to chronic fetal vasopressin infusion. American Physiological Society 1985 E287 - E291).

Die Veröffentlichung von Corpening J W et al. Ingested bovine amniotic fluid enhances morphine antinociception in rats. Physiology & Behavior 2000 (70) 15-18 betrifft die Verabreichung von Rinderfruchtwasser an Ratten.

Bei der Verwendung von Fruchtwasser von Rindern bei einer Amnio-Infusion bei anderen Säugetieren, wie z. B. Ratten, besteht jedoch ein erhebliches Risiko, dass Erbmaterial der Rinder in die DNA des Föten eingebaut und sogenannte Chimäre gebildet werden. Daher ist die Verwendung von Rinderfruchtwasser bei einer Amnio-Infusion bei anderen Säugetieren, insbesondere bei Menschen, nicht sinnvoll, zumal die Herstellung von Chimären in den meisten Ländern strafbar ist.

Die Publikation von Doi S et al. Effect of Maternal Hydration on Oligohydramnios: A Comparison of Three Volume Expansion Methods. Obstetrics & Gynecology 1998, 92 (4), 525-529 untersucht den Einfluss einer intravenösen Verabreichung von einer isotonischen Flüssigkeit oder von einer hypotonischen Flüssigkeit sowie einer oralen Verabreichung von Wasser auf den Amniotic Fluid Index (AFI) bei Frauen. Als hypotonische Lösung wird Ringer-Lösung verwendet. Diese weist eine viel zu hohe Chloridionenkonzentration mit den damit verbundenen Nachteilen auf (siehe oben).

Die Patentanmeldung EP 1 719 517 A1 betrifft eine künstliche physiologische Kochsalzlösung mit einer Osmolarität im Bereich von 260 mOsm/l bis 320 mOsm/l, die vorzugsweise nicht mehr als 200 mmol/l Chloridionen enthält. Die Chloridionenkonzentration ist jedoch viel höher als vorliegend gefordert. Bzgl. der Nachteile einer zu hohen Chloridionenkonzentration wird auf die vorstehenden Erläuterungen verwiesen.

Die Patentanmeldung EP 0 998 916 A1 beschäftigt sich mit medizinischen Zusammensetzungen für Applikationen über die Schleimhaut. Die Zusammensetzungen sollen eine Osmolarität kleiner 290 mOsm/l aufweisen. Das einzige Beispiel mit einer Osmolarität im vorliegend geforderten Bereich weist allerdings eine zu hohe Chloridionenkonzentration auf. Bzgl. der Nachteile einer zu hohen Chloridionenkonzentration wird wiederum auf die vorstehenden Erläuterungen verwiesen.

In Anbetracht des Standes der Technik war es daher Aufgabe der vorliegenden Erfindung, bessere Möglichkeiten zur Behandlung von Patientinnen mit vorzeitigem Blasensprung (PPROM) aufzuzeigen. Gewünscht wurde insbesondere eine Lösung für die Nachteile und Probleme der bisherigen Amnioninfusionsverfahren. So sollten vor allem Wege aufgezeigt werden, wie gesundheitliche Risiken für den Fötus, insbesondere das Risiko einer Frühgeburt, möglichst vermieden werden können. Weiterhin wurde eine möglichst rasche und möglichst gute Lungenreifung gewünscht, um die Überlebenschancen des Fötus, selbst bei einer Frühgeburt, zu erhöhen. Darüber hinaus sollte eine Schädigung von Organen des Fötus, vor allem seiner Haut, seiner Augen und seiner Verdauungsorgane, insbesondere seiner Nieren, möglichst vermieden werden. Wichtig war in diesem Zusammenhang auch, eine Änderung der fetalen Programmierung des Fötus nach Möglichkeit zu vermeiden. Schließlich sollte sich die erfindungsgemäße Lösung auch auf vergleichbar einfache Art und Weise kostengünstig realisieren lassen und aus moralischer, ethischer sowie rechtlicher Sicht unbedenklich sein.

Gemäß einem ersten Aspekt der vorliegenden Erfindung werden diese sowie weitere Aufgaben, die sich aus den in dieser Anmeldung diskutierten Zusammenhängen in naheliegender Weise ergeben, durch eine für medizinische Anwendungen bisher unbekannte wässrige Zusammensetzung mit allen Merkmalen des vorliegenden Patentanspruchs 1 gelöst.

Gemäß einem weiteren Aspekt der vorliegenden Erfindung werden diese sowie weitere Aufgaben, die sich aus den in dieser Anmeldung diskutierten Zusammenhängen in naheliegender Weise ergeben, durch eine zur Anwendung bei einer Amnioninfusion bisher unbekannte wässrige Zusammensetzung mit allen Merkmalen des vorliegenden Patentanspruchs 14 gelöst.

Die auf den Patentanspruch 1 rückbezogenen Unteransprüche beschreiben besonders vorteilhafte Ausführungsformen der wässrigen Zusammensetzung. Darüber hinaus wird eine besonders vorteilhafte Zusammenstellung zur Herstellung der erfindungsgemäßen Zusammensetzung in einem unabhängigen Patentanspruch beansprucht.

Sowohl die Bereitstellung einer bisher für medizinische Anwendungen unbekannten wässrigen Zusammensetzung, die osmotisch aktive Teilchen umfasst und weniger als 70 µl⁻¹ Amnionzellen enthält, wobei die Osmolarität der Zusammensetzung im Bereich von 240,0 mosm/l bis kleiner 308,0 mosm/l liegt und wobei die Chloridionenkonzentration in der Zusammensetzung höchstens 130,0 mmol/l ist, als auch die Bereitstellung einer bisher zur Anwendung bei einer Amnioninfusion unbekannten wässrigen Zusammensetzung, die weniger als 70 µl⁻¹ Amnionzellen und mindestens ein Surfactant enthält, erlaubt eine bessere Behandlung von Patientinnen mit vorzeitigem Blasensprung (PPROM).

Dabei werden insbesondere die Nachteile und Probleme der bisherigen Amnioninfusionsverfahren überwunden. So werden Möglichkeiten aufgezeigt, wie gesundheitliche Risiken für den Fötus, insbesondere das Risiko einer Frühgeburt, möglichst vermieden werden können. Weiterhin wird eine vergleichsweise schnelle und gute Lungenreifung gewährleistet, welches wiederum die Überlebenschancen des Fötus, selbst bei einer Frühgeburt, deutlich erhöht. Darüber hinaus wird insbesondere bei der Variante nach Anspruch 1 eine Schädigung von Organen des Fötus, vor allem seiner Haut, seiner Augen und seiner Verdauungsorgane, insbesondere seiner Nieren, bestmöglich vermieden. Das Risiko einer Änderung der fetalen Programmierung des Fötus wird insbesondere bei der Variante nach Anspruch 1 bestmöglich reduziert. Schließlich kann die erfindungsgemäße Lösung auch auf vergleichbar einfache Art und Weise kostengünstig realisiert werden und ist aus moralischer, ethischer sowie rechtlicher Sicht absolut unbedenklich.

Die vorliegende Erfindung betrifft gemäß einem ersten Aspekt eine wässrige Zusammensetzung zur Anwendung in einem Verfahren zur chirurgischen oder therapeutischen Behandlung des menschlichen oder tierischen Körpers oder in einem Diagnostizierverfahren, das am menschlichen oder tierischen Körper vorgenommen wird. Verfahren zur therapeutischen Behandlung, insbesondere des menschlichen Körpers, stehen dabei besonders im Vordergrund. Ein besonders bevorzugtes Anwendungsgebiet ist die Amnioninfusion, vor allem die Langzeit-Amnioninfusion, bevorzugt bei Menschen, insbesondere zur Behandlung von Patientinnen mit vorzeitigem Blasensprung (PPROM), vor allem von Schwangeren im zweiten Trimester (13. Woche bis 24. Woche) und dritten Trimester (25. Woche bis 40. Woche), insbesondere in der 16. bis 34. Woche. Weiterhin ist ein Einsatz dieser erfindungsgemäßen Zusammensetzung für alle medizinischen Anwendungen vorteilhaft, bei denen eine vorzugsweise hypotone wässrige Zusammensetzung eingesetzt wird. Besonders bevorzugte Anwendungsgebiete schließen das Spülen von Körperhöhlen, bevorzugt das Ausspülen von eitrigen Höhlen, insbesondere in der Lunge, wie z. B. im Rahmen einer bronchoalveolären Lavage, sowie das Spülen von mit Häuten, insbesondere mit Schleimhäuten, überzogenen Körperbereichen ein. Dabei ist die Anwendung der erfindungsgemäßen Zusammensetzung insbesondere für die Behandlung von Säuglingen und Kleinkindern mit einem Alter kleiner 3 Jahre, bevorzugt kleiner 2 Jahre, zweckmäßigerweise kleiner 1 Jahr, besonders bevorzugt kleiner 6 Monate, insbesondere kleiner 3 Monate, besonders vorteilhaft.

Die Osmolarität dieser erfindungsgemäßen Zusammensetzung ist kleiner als die von physiologischer Kochsalzlösung und somit kleiner 308,0 mosm/l. Bevorzugt ist sie kleiner 307,0 mosm/l, zweckmäßigerweise kleiner 305,0 mosm/l, besonders bevorzugt kleiner 303,0 mosm/l, zweckmäßigerweise höchstens 300,0 mosm/l, günstigerweise höchstens 290,0 mosm/l, insbesondere höchstens 280,0 mosm/l.

Weiterhin ist die Osmolarität dieser erfindungsgemäßen Zusammensetzung mindestens 240,0 mosm/l, bevorzugt mindestens 250,0 mosm/l, insbesondere mindestens 260,0 mosm/l, um eine Lyse von Zellen nach Möglichkeit zu vermeiden.

Besonders bevorzugt liegt die Osmolarität dieser erfindungsgemäßen Zusammensetzung im Bereich von 240,0 mosm/l bis 300,0 mosm/l, bevorzugt im Bereich von 250,0 mosm/l bis 290,0 mosm/l, insbesondere im Bereich von 260,0 mosm/l bis 280,0 mosm/l.

Die Chloridionenkonzentration in dieser erfindungsgemäßen Zusammensetzung ist höchstens 130,0 mmol/l und somit kleiner als die in physiologischer Kochsalzlösung sowie in den vorstehend genannten Ringer-Lösungen. Bevorzugt ist sie kleiner 130,0 mmol/l, besonders bevorzugt kleiner 125,0 mmol/l, insbesondere höchstens 120,0 mmol/l.

Weiterhin ist die Chloridionenkonzentration in dieser erfindungsgemäßen Zusammensetzung vorzugsweise mindestens 90,0 mmol/l, bevorzugt mindestens 100,0 mmol/l, insbesondere mindestens 105,0 mmol/l.

Besonders bevorzugt liegt die Chloridionenkonzentration in dieser erfindungsgemäßen Zusammensetzung im Bereich von 100,0 mmol/l bis 120,0 mmol/l, insbesondere im Bereich von 105,0 mmol/l bis 115,0 mmol/l.

Die Natriumionenkonzentration in dieser erfindungsgemäßen Zusammensetzung ist vorzugsweise kleiner als die in physiologischer Kochsalzlösung und somit vorzugsweise kleiner 154,0 mmol/l. Bevorzugt ist sie kleiner 150,0 mmol/l, insbesondere höchstens 145,0 mmol/l. Andererseits ist sie vorzugsweise größer 125,0 mmol/l, bevorzugt mindestens 130,0 mmol/l, und liegt zweckmäßigerweise im Bereich von 130,0 mmol/l bis 145,0 mmol/l, insbesondere im Bereich von 130,0 mmol/l bis 140,0 mmol/l.

Die Kaliumionenkonzentration in dieser erfindungsgemäßen Zusammensetzung liegt vorzugsweise im Bereich von 3,5 mmol/l bis 5,2 mmol/l, insbesondere im Bereich von 3,5 mmol/l bis 4,5 mmol/l.

Die Calciumionenkonzentration in dieser erfindungsgemäßen Zusammensetzung ist zweckmäßigerweise kleiner 8,0 mmol/l, bevorzugt kleiner 6,0 mmol/l, besonders bevorzugt kleiner 4,0 mmol/l, und liegt vorzugsweise im Bereich von 1,0 mmol/l bis 3,0 mmol, bevorzugt im Bereich von 1,0 mmol/l bis 2,5 mmol/l, insbesondere im Bereich von 1,5 mmol/l bis kleiner 2,3 mmol/l.

Die Magnesiumionenkonzentration in dieser erfindungsgemäßen Zusammensetzung liegt vorzugsweise im Bereich von 0,0 mmol/l bis 2,0 mmol/l, bevorzugt im Bereich von 0,1 mmol/l bis 1,5 mmol/l, besonders bevorzugt im Bereich von 0,1 mmol/l bis 1,0 mmol/l, insbesondere im Bereich von 0,1 mmol/l bis kleiner 0,8 mmol/l.

Die Harnstoffkonzentration in dieser erfindungsgemäßen Zusammensetzung liegt vorzugsweise im Bereich von 0,0 mg/dl bis 10,0 mg/dl.

Die Harnsäurekonzentration in dieser erfindungsgemäßen Zusammensetzung liegt vorzugsweise im Bereich von 0,0 mg/dl bis 5,0 mg/dl.

Die Phosphationenkonzentration in dieser erfindungsgemäßen Zusammensetzung liegt vorzugsweise im Bereich von 0,0 mg/dl bis 8,0 mg/dl, insbesondere im Bereich von 0,1 mg/dl bis 7,0 mg/dl.

Die Glucosekonzentration in dieser erfindungsgemäßen Zusammensetzung liegt vorzugsweise im Bereich von 0,0 mg/dl bis 340,0 mg/dl. Sie ist bevorzugt kleiner 300,0 mg/dl, günstigerweise kleiner 200,0 mg/dl, zweckmäßigerweise kleiner 100,0 mg/dl, insbesondere kleiner 60,0 mg/dl. Im Rahmen einer ganz besonders bevorzugten Ausführungsform der vorliegenden Erfindung enthält die Zusammensetzung weniger als 50,0 mg/dl, günstigerweise weniger als 25,0 mg/dl, zweckmäßigerweise weniger als 10,0 mg/dl, insbesondere keine, Glucose. Die Konzentration der Laktatsalze in dieser erfindungsgemäßen Zusammensetzung liegt vorzugsweise im Bereich von 0,0 mmol/l bis 20,0 mmol/l.

Die Konzentration der Citratsalze in dieser erfindungsgemäßen Zusammensetzung liegt vorzugsweise im Bereich von 0,0 mg/l bis 100,0 mg/l.

Die Hydrogencarbonationenkonzentration in dieser erfindungsgemäßen Zusammensetzung liegt vorzugsweise im Bereich von 5,0 mmol/l bis 100,0 mmol/l, bevorzugt im Bereich von 5,0 mmol/l bis 50,0 mmol/l, günstigerweise im Bereich von 5,0 mmol/l bis kleiner 24,0 mmol/l, insbesondere im Bereich von 10,0 mmol/l bis 20,0 mmol/l.

Die Gesamt-Eiweißkonzentration in dieser erfindungsgemäßen Zusammensetzung liegt vorzugsweise im Bereich von 0,0 g/l bis 10,0 g/l.

Die Albuminkonzentration in dieser erfindungsgemäßen Zusammensetzung liegt vorzugsweise im Bereich von 0,0 g/dl bis 5,0 g/dl, insbesondere im Bereich von 0,0 g/dl bis kleiner 2,0 g/dl.

Die Kupferionenkonzentration in dieser erfindungsgemäßen Zusammensetzung liegt vorzugsweise im Bereich von 0,0 µg/dl bis 150,0 µg/dl, bevorzugt im Bereich von 1,0 µg/dl bis 100,0 µg/dl, insbesondere im Bereich von 5,0 µg/dl bis 50,0 µg/dl.

Die Selenionenkonzentration in dieser erfindungsgemäßen Zusammensetzung liegt vorzugsweise im Bereich von 0,0 µg/dl bis 50,0 µg/dl, bevorzugt im Bereich von 1,0 µg/dl bis 25,0 µg/dl, insbesondere im Bereich von 2,0 µg/dl bis 15,0 µg/dl.

Die Zinkionenkonzentration in dieser erfindungsgemäßen Zusammensetzung liegt vorzugsweise im Bereich von 0,0 µg/dl bis 30,0 µg/dl, bevorzugt im Bereich von 5,0 µg/dl bis 25,0 µg/dl, insbesondere im Bereich von 10,0 µg/dl bis 24,0 µg/dl.

Im Rahmen der vorliegenden Erfindung wurde festgestellt, dass ein Zusatz von mindestens einem Surfactant zu den erfindungsgemäßen wässrigen Zusammensetzungen, insbesondere für die Lungenreifung des Fötus bei der Amnioninfusion, besonders vorteilhaft ist.

Dementsprechend stellt die vorliegende Erfindung eine wässrige Zusammensetzung zur Anwendung bei einer Amnioninfusion unter Schutz, welche weniger als 70 µl⁻¹ Amnionzellen und mindestens ein Surfactant, insbesondere mindestens ein Phospholipid und/oder mindestens ein Surfactant-Protein, enthält. Bzgl. der bevorzugten Anwendungsgebiete dieser Zusammensetzung, der bevorzugten Komponenten dieser Zusammensetzung, der bevorzugten Konzentrationen dieser Komponenten und der Osmolarität gilt im Prinzip das zuvor und das im folgenden gesagte, mit der Ausnahme, dass die vorgegebene Osmolarität und die vorgegebene Chloridionenkonzentration unter diesen Umständen nicht zwingend erfüllt sein müssen.

Die erfindungsgemäße Zusammensetzung gemäß Anspruch 1 enthält zweckmäßigerweise ebenfalls mindestens einen Surfactant.

Der Begriff "Surfactant" bezeichnet im Rahmen der vorliegenden Erfindung oberflächenaktive Substanzen, die in der menschlichen Lunge zu finden sind. Diese werden normalerweise von spezialisierten Lungenzellen (Pneumozyten Typ II) gebildet und schließen insbesondere Phospholipide und Surfactant-Proteine ein.

Im Rahmen einer ersten besonders bevorzugten Ausführungsform der vorliegenden Erfindung enthält die erfindungsgemäße Zusammensetzung gemäß Anspruch 1 mindestens ein Phospholipid. Im Rahmen einer zweiten besonders bevorzugten Ausführungsform der vorliegenden Erfindung enthält die erfindungsgemäße Zusammensetzung gemäß Anspruch 1 mindestens ein Surfactant-Protein. Im Rahmen einer weiteren besonders bevorzugten Ausführungsform der vorliegenden Erfindung enthält die erfindungsgemäße Zusammensetzung gemäß Anspruch 1 mindestens ein Phospholipid und mindestens ein Surfactant-Protein.

Erfindungsgemäß besonders bevorzugte Phospholipide (Phosphatide) schließen fettähnliche Triglyceride ein, die zwei langkettige Fettsäuren und ein Phosphorsäurerest, an den vorzugsweise noch eine Base gebunden ist, enthalten. Ganz besonders bevorzugt werden solche Verbindungen, die in tierischen und pflanzlichen Zellen, vor allem im Gehirn, im Herzen, in der Leber, im Eidotter sowie in der Sojabohne, vorkommen.

Für die Zwecke der vorliegenden Erfindung ganz besonders vorteilhafte Phospholipide schließen O-Phosphatidyl-ethanolamin und O-Phosphatidylcholin ein. Die Stereochemie dieser Verbindungen unterliegt dabei keinen besonderen Beschränkungen. Besonders günstig ist jedoch die Verwendung des 1-sn-Phosphatidylethanolamins und des 3-sn-Phosphatidylcholins, insbesondere des 2,3-Diacyl-sn-glycero-1-Phosphoethanolamins (früher Kephalin genannt) und des 1,2-Diacyl-sn-glycero-3-Phosphocholins (früher Lecithin genannt). Der Einsatz von O-Phosphatidylcholin, bevorzugt von 3-sn-Phosphatidylcholin, insbesondere von 1,2-Diacyl-sn-glycero-3-Phosphocholin hat sich dabei ganz besonders bewährt, wobei Dipalmitoylphosphatidylcholine besonders bevorzugt werden.

Sphingolipide sind eine weitere Klasse erfindungsgemäß besonders bevorzugter Phospholipide. Hierzu gehören insbesondere Verbindungen, bei denen das Glycerin durch einen ungesättigten Aminoalkohol, bevorzugt Sphingosin, ersetzt ist. Sphingosinphosphate werden üblicherweise auch als Sphingomyelin bezeichnet.

Im Rahmen einer besonders bevorzugten Variante der vorliegenden Erfindung enthalten die erfindungsgemäße Zusammensetzungen ein Gemisch aus Phosphatiden, das neben dem mindestens einen Phospholipid eine oder mehrere gesättigte oder ungesättigte Fettsäuren, besonders bevorzugt Palmitin-, Stearin-, Öl-, Linol- und/oder Linolensäure, enthält.

Das Gewichtsverhältnis von Phospholipid zu Surfactant-Protein liegt in den erfindungsgemäßen Zusammensetzungen günstigerweise im Bereich von 5:1 bis 15:1, bevorzugt im Bereich von 8:1 bis 12:1, insbesondere im Bereich von 9:1 bis 11:1.

Für die Zwecke der vorliegenden Erfindung besonders bevorzugte Surfactant-Proteine umfassen biophysische Surfactant-Proteine, insbesondere Surfactant-Protein-B und Surfactant-Protein-C, immunologische Surfactant-Proteine, insbesondere Surfactant-Protein-A und Surfactant-Protein-D, sowie regulatorische Surfactant-Proteine.

Die Gesamtlipidkonzentration in den erfindungsgemäßen Zusammensetzungen liegt vorzugsweise im Bereich von 0,0 mg/l bis 2000,0 mg/l, bevorzugt im Bereich von 1,0 mg/l bis 1500,0 mg/l, besonders bevorzugt im Bereich von 25,0 mg/l bis 1000,0 mg/l, insbesondere im Bereich von 50,0 mg/l bis 500,0 mg/l.

Die Phosphatidylcholinkonzentration in den erfindungsgemäßen Zusammensetzungen liegt vorzugsweise im Bereich von 0,0 mg/l bis 1000,0 mg/l, bevorzugt im Bereich von 1,0 mg/l bis 500,0 mg/l, besonders bevorzugt im Bereich von 10,0 mg/l bis 250,0 mg/l, insbesondere im Bereich von 50,0 mg/l bis 150,0 mg/l. Der Gewichtsanteil von Phosphatidylcholin, bezogen auf alle Lipide, ist vorzugsweise größer 50,0 Gew.-%, bevorzugt größer 70,0 Gew.-% und liegt ganz besonders bevorzugt im Bereich von 75,0 Gew.-% bis 85,0 Gew.-%.

Die Dipalmitoylphosphatidylcholinkonzentration in den erfindungsgemäßen Zusammensetzungen liegt vorzugsweise im Bereich von 0,0 mg/l bis 600,0 mg/l, bevorzugt im Bereich von 1,0 mg/l bis 400,0 mg/l, besonders bevorzugt im Bereich von 10,0 mg/l bis 250,0 mg/l, insbesondere im Bereich von 20,0 mg/l bis 150,0 mg/l. Der Gewichtsanteil von Dipalmitoylphosphatidylcholin, bezogen auf Phosphatidylcholin, liegt vorzugsweise im Bereich von 36,0 Gew.-% bis 60,0 Gew.-%.

Die Palmitoylmyristolphosphatidylcholinkonzentration in den erfindungsgemäßen Zusammensetzungen liegt vorzugsweise im Bereich von 0,0 mg/l bis 200,0 mg/l, bevorzugt im Bereich von 1,0 mg/l bis 100,0 mg/l, besonders bevorzugt im Bereich von 2,0 mg/l bis 50,0 mg/l, insbesondere im Bereich von 2,5 mg/l bis 10,0 mg/l.

Die Phosphatidylglyzerinkonzentration in den erfindungsgemäßen Zusammensetzungen liegt vorzugsweise im Bereich von 0,0 mg/l bis 200,0 mg/l, bevorzugt im Bereich von 1,0 mg/l bis 100,0 mg/l, besonders bevorzugt im Bereich von 2,0 mg/l bis 50,0 mg/l, insbesondere im Bereich von 5,0 mg/l bis 20,0 mg/l. Der Gewichtsanteil von Phosphatidylglyzerin, bezogen auf Phosphatidylcholin, liegt vorzugsweise im Bereich von 5,0 Gew.-% bis 20,0 Gew.-%, insbesondere im Bereich von 10,0 Gew.-% bis 15,0 Gew.-%.

Die Phosphatidylethanolaminkonzentration in den erfindungsgemäßen Zusammensetzungen liegt vorzugsweise im Bereich von 0,0 mg/l bis 100,0 mg/l, bevorzugt im Bereich von 1,0 mg/l bis 50,0 mg/l, besonders bevorzugt im Bereich von 2,0 mg/l bis 25,0 mg/l, insbesondere im Bereich von 3,0 mg/l bis 10,0 mg/l. Der Gewichtsanteil von Phosphatidylethanolamin, bezogen auf Phosphatidylcholin, liegt vorzugsweise im Bereich von 1,0 Gew.-% bis 10,0 Gew.- %, insbesondere im Bereich von 3,0 Gew.-% bis 7,0 Gew.-%.

Die Phosphatidylinositolkonzentration in den erfindungsgemäßen Zusammensetzungen liegt vorzugsweise im Bereich von 0,0 mg/l bis 70,0 mg/l, bevorzugt im Bereich von 1,0 mg/l bis 50,0 mg/l, besonders bevorzugt im Bereich von 1,5 mg/l bis 25,0 mg/l, insbesondere im Bereich von 2,0 mg/l bis 10,0 mg/l. Der Gewichtsanteil von Phosphatidylinositol, bezogen auf Phosphatidylcholin, liegt vorzugsweise im Bereich von 1,0 Gew.-% bis 10,0 Gew.-%, insbesondere im Bereich von 2,0 Gew.-% bis 6,0 Gew.-%.

Die Phosphatidylserinkonzentration in den erfindungsgemäßen Zusammensetzungen liegt vorzugsweise im Bereich von 0,0 mg/l bis 50,0 mg/l, bevorzugt im Bereich von 0,1 mg/l bis 25,0 mg/l, besonders bevorzugt im Bereich von 0,2 mg/l bis 10,0 mg/l, insbesondere im Bereich von 0,5 mg/l bis 5,0 mg/l. Der Gewichtsanteil von Phosphatidylserin, bezogen auf Phosphatidylcholin, liegt vorzugsweise im Bereich von 0,1 Gew.-% bis 10,0 Gew.-%, insbesondere im Bereich von 0,5 Gew.-% bis 2,5 Gew.-%.

Die Sphingomyelinkonzentration in den erfindungsgemäßen Zusammensetzungen liegt vorzugsweise im Bereich von 0,0 mg/l bis 1000,0 mg/l, bevorzugt im Bereich von 1,0 mg/l bis 500,0 mg/l, besonders bevorzugt im Bereich von 5,0 mg/l bis 100,0 mg/l, insbesondere im Bereich von 10,0 mg/l bis 40,0 mg/l. Zweckmäßigerweise wird sie analog zum natürlichen Fruchtwasser an das Alter des Fötus angepasst, wobei bei jüngeren Föten eine niedrigere Konzentration und bei älteren Föten eine höhere Konzentration gewählt wird.

Die Lysophospholipidkonzentration in den erfindungsgemäßen Zusammensetzungen liegt vorzugsweise im Bereich von 0,0 mg/l bis 10,0 mg/l, bevorzugt im Bereich von 0,1 mg/l bis 5,0 mg/l, besonders bevorzugt im Bereich von 0,2 mg/l bis 2,5 mg/l, insbesondere im Bereich von 0,3 mg/l bis 1,0 mg/l. Der Gewichtsanteil von Lysophospholipid, bezogen auf Phosphatidylcholin, liegt vorzugsweise im Bereich von 0,1 Gew.-% bis 5,0 Gew.-% und ist bevorzugt kleiner als 1,0 Gew.-%.

Das Gewichtsverhältnis von Lecithin zu Sphingomyelin in den erfindungsgemäßen Zusammensetzungen ist vorzugsweise größer 1,5; bevorzugt größer 1,75; insbesondere größer 2,0.

Die Cholesterolkonzentration in den erfindungsgemäßen Zusammensetzungen liegt vorzugsweise im Bereich von 0,0 mg/l bis 100,0 mg/l, bevorzugt im Bereich von 1,0 mg/l bis 50,0 mg/l, besonders bevorzugt im Bereich von 2,0 mg/l bis 25,0 mg/l, insbesondere im Bereich von 3,0 mg/l bis 10,0 mg/l. Der Gewichtsanteil von Cholesterol, bezogen auf alle Lipide, liegt vorzugsweise im Bereich von 1,0 Gew.- % bis 20,0 Gew.-%, insbesondere im Bereich von 5,0 Gew.-% bis 10,0 Gew.-%.

Die Surfactant Protein A-Konzentration in den erfindungsgemäßen Zusammensetzungen liegt vorzugsweise im Bereich von 0,0 mg/l bis 500,0 mg/l, bevorzugt im Bereich von 1,0 mg/l bis 100,0 mg/l, besonders bevorzugt im Bereich von 2,0 mg/l bis 50,0 mg/l, insbesondere im Bereich von 3,0 mg/l bis 10,0 mg/l. Die Surfactant Protein B-Konzentration in den erfindungsgemäßen Zusammensetzungen liegt vorzugsweise im Bereich von 0,0 mg/l bis 100,0 mg/l, bevorzugt im Bereich von 0,1 mg/l bis 50,0 mg/l, besonders bevorzugt im Bereich von 0,2 mg/l bis 10,0 mg/l, insbesondere im Bereich von 0,3 mg/l bis 2,0 mg/l.

Die Surfactant Protein D-Konzentration in den erfindungsgemäßen Zusammensetzungen liegt vorzugsweise im Bereich von 0,0 µg/l bis 1000,0 µg/l, bevorzugt im Bereich von 1,0 mg/l bis 500,0 mg/l, besonders bevorzugt im Bereich von 2,0 mg/l bis 100,0 mg/l, insbesondere im Bereich von 3,0 mg/l bis 25,0 mg/l.

Der pH-Wert der erfindungsgemäßen Zusammensetzungen, gemessen bei 20°C, liegt vorzugsweise im Bereich von 6,5 bis 9,0, bevorzugt im Bereich von 7,0 bis 8,6, insbesondere im Bereich von 7,5 bis 8,5.

Die Apoprotein B-Konzentration in der erfindungsgemäßen Zusammensetzungen liegt vorzugsweise im Bereich von 0,0 g/l bis 1,0 g/l, bevorzugt im Bereich von 0,01 g/l bis 0,5 g/l, besonders bevorzugt im Bereich von 0,02 g/l bis 0,3 g/l, insbesondere im Bereich von 0,05 g/l bis 0,2 g/l.

Die Lipoproteinkonzentration in der erfindungsgemäßen Zusammensetzungen liegt vorzugsweise im Bereich von 0,0 mg/l bis 1000,0 mg/l, bevorzugt im Bereich von 1,0 mg/l bis 500,0 mg/l, besonders bevorzugt im Bereich von 5,0 mg/l bis 250,0 mg/l, insbesondere im Bereich von 50,0 mg/l bis 125,0 mg/l.

Im Rahmen einer besonders bevorzugten Variante werden die erfindungsgemäßen Zusammensetzungen dazu genutzt, die Lungenreifung des Fötus zu beschleunigen, um seine Überlebenschancen, insbesondere bei einer Frühgeburt, weiter zu erhöhen. Zu diesem Zweck wird die Konzentration mindestens eines Surfactants, bevorzugt mindestens eines Phospholipids und/oder mindestens eines Surfactant-Proteins, insbesondere mindestens eines Phospholipids, im Fruchtwasser erhöht. Dies kann insbesondere durch Amnioninfusion einer wässrigen Zusammensetzung erfolgen, das eine höhere Konzentration des mindestens einen Surfactants als das natürliche Fruchtwasser aufweist.

Besonders bewährt haben sich in diesem Zusammenhang Zusammensetzungen mit einem Gesamtlipidanteil aller Phospholipide größer 120,0 mg/l, bevorzugt größer 140,0 mg/l, besonders bevorzugt größer 160,0 mg/l, günstigerweise größer 175,0 mg/l, vorteilhafterweise größer 200,0 mg/l, ganz besonders bevorzugt größer 250,0 mg/l, insbesondere größer 400,0 mg/l.

Die Phosphatidylcholinkonzentration bevorzugter Zusammensetzungen ist vorzugsweise größer 120,0 mg/l, bevorzugt größer 140,0 mg/l, besonders bevorzugt größer 160,0 mg/l, günstigerweise größer 175,0 mg/l, vorteilhafterweise größer 200,0 mg/l, ganz besonders bevorzugt größer 250,0 mg/l, insbesondere größer 400,0 mg/l.

Die Dipalmitoylphosphatidylcholinkonzentration bevorzugter Zusammensetzungen ist vorzugsweise größer 50,0 mg/l, bevorzugt größer 60,0 mg/l, besonders bevorzugt größer 75,0 mg/l, günstigerweise größer 100,0 mg/l, vorteilhafterweise größer 125,0 mg/l, ganz besonders bevorzugt größer 150,0 mg/l, insbesondere größer 200,0 mg/l.

Die Palmitoylmyristolphosphatidylcholinkonzentration bevorzugter Zusammensetzungen ist vorzugsweise größer 7,0 mg/l, bevorzugt größer 10,0 mg/l, besonders bevorzugt größer 15,0 mg/l, günstigerweise größer 20,0 mg/l, ganz besonders bevorzugt größer 25,0 mg/l, insbesondere größer 30,0 mg/l.

Die Phosphatidylglyzerinkonzentration bevorzugter Zusammensetzungen ist vorzugsweise größer 12,0 mg/l, bevorzugt größer 15,0 mg/l, besonders bevorzugt größer 20,0 mg/l, günstigerweise größer 25,0 mg/l, vorteilhafterweise größer 30,0 mg/l, ganz besonders bevorzugt größer 40,0 mg/l, insbesondere größer 50,0 mg/l.

Die Phosphatidylethanolaminkonzentration bevorzugter Zusammensetzungen ist vorzugsweise größer 6,0 mg/l, bevorzugt größer 8,0 mg/l, besonders bevorzugt größer 10,0 mg/l, günstigerweise größer 12,5 mg/l, vorteilhafterweise größer 15,0 mg/l, ganz besonders bevorzugt größer 17,5 mg/l, insbesondere größer 20,0 mg/l.

Die Phosphatidylinositolkonzentration bevorzugter Zusammensetzungen ist vorzugsweise größer 6,0 mg/l, bevorzugt größer 8,0 mg/l, besonders bevorzugt größer 10,0 mg/l, günstigerweise größer 12,5 mg/l, vorteilhafterweise größer 15,0 mg/l, ganz besonders bevorzugt größer 17,5 mg/l, insbesondere mindestens 20,0 mg/l.

Die Phosphatidylserinkonzentration bevorzugter Zusammensetzungen ist vorzugsweise größer 1,5 mg/l, bevorzugt größer 1,75 mg/l, besonders bevorzugt größer 2,0 mg/l, günstigerweise größer 3,0 mg/l, vorteilhafterweise größer 4,0 mg/l, ganz besonders bevorzugt größer 5,0 mg/l, insbesondere größer 6,0 mg/l.

Die Sphingomyelinkonzentration bevorzugter Zusammensetzungen ist vorzugsweise größer 10,0 mg/l, bevorzugt größer 25,0 mg/l, besonders bevorzugt größer 40,0 mg/l, günstigerweise größer 50,0 mg/l, vorteilhafterweise größer 60,0 mg/l, ganz besonders bevorzugt größer 75,0 mg/l, insbesondere größer 400 mg/l.

Die Lysophospholipidkonzentration bevorzugter Zusammensetzungen ist vorzugsweise größer 0,6 mg/l, bevorzugt größer 0,8 mg/l, besonders bevorzugt größer 1,0 mg/l, günstigerweise größer 1,25 mg/l, vorteilhafterweise größer 1,5 mg/l, ganz besonders bevorzugt größer 1,75 mg/l, insbesondere größer 2,0 mg/l.

Die Cholesterolkonzentration bevorzugter Zusammensetzungen ist vorzugsweise größer 6,0 mg/l, bevorzugt größer 8,0 mg/l, besonders bevorzugt größer 10,0 mg/l, günstigerweise größer 12,5 mg/l, vorteilhafterweise größer 15,0 mg/l, ganz besonders bevorzugt größer 20,0 mg/l, insbesondere mindestens 25,0 mg/l.

Die Surfactant Protein A-Konzentration bevorzugter Zusammensetzungen ist vorzugsweise größer 6,0 mg/l, bevorzugt größer 8,0 mg/l, besonders bevorzugt größer 10,0 mg/l, günstigerweise größer 12,5 mg/l, vorteilhafterweise größer 15,0 mg/l, ganz besonders bevorzugt größer 20,0 mg/l, insbesondere größer 25,0 mg/l.

Die Surfactant Protein B-Konzentration bevorzugter Zusammensetzungen ist vorzugsweise größer 0,6 mg/l, bevorzugt größer 0,8 mg/l, besonders bevorzugt größer 1,0 mg/l, günstigerweise größer 1,25 mg/l, vorteilhafterweise größer 1,5 mg/l, ganz besonders bevorzugt größer 2,0 mg/l, insbesondere größer 2,5 mg/l.

Die Surfactant Protein D-Konzentration bevorzugter Zusammensetzungen ist vorzugsweise größer 12,0 mg/l, bevorzugt größer 15,0 mg/l, besonders bevorzugt größer 20,0 mg/l, günstigerweise größer 25,0 mg/l, vorteilhafterweise größer 30,0 mg/l, ganz besonders bevorzugt größer 40,0 mg/l, insbesondere größer 50,0 mg/l.

Die Apoprotein B-Konzentration bevorzugter Zusammensetzungen ist vorzugsweise größer 0,15 mg/l, bevorzugt größer 0,175 mg/l, besonders bevorzugt größer 0,20 mg/l, günstigerweise größer 0,25 mg/l, vorteilhafterweise größer 0,30 mg/l, ganz besonders bevorzugt größer 0,40 mg/l, insbesondere größer 0,55 mg/l.

Die Lipoproteinkonzentration bevorzugter Zusammensetzungen ist vorzugsweise größer 85,0 mg/l, bevorzugt größer 90,0 mg/l, besonders bevorzugt größer 100,0 mg/l, günstigerweise größer 125,0 mg/l, vorteilhafterweise größer 175,0 mg/l, ganz besonders bevorzugt größer 250,0 mg/l, insbesondere größer 400,0 mg/l.

Weiterhin enthalten die erfindungsgemäßen Zusammensetzungen Wasser. Dessen Anteil beträgt vorzugsweise mindestens 50,0 Gew.-%, bevorzugt mindestens 70,0 Gew.-%, insbesondere mindestens 90,0 Gew.-%, jeweils bezogen auf das Gesamtgewicht der Zusammensetzung. Im Rahmen einer besonders bevorzugten Variante der vorliegenden Erfindung enthalten die erfindungsgemäßen Zusammensetzungen keine weiteren Lösungsmittel.

Der Anteil von weiteren Komponenten als den in dieser Anmeldung explizit genannten essentiellen und optionalen Bestandteilen ist vorzugsweise kleiner 5,0 Gew.-%, bevorzugt kleiner 2,5 Gew.-%, besonders bevorzugt kleiner 1,0 Gew.-%, insbesondere kleiner 0,5 Gew.-%, jeweils bezogen auf das Gesamtgewicht der Zusammensetzung. Im Rahmen einer besonders vorteilhaften Variante der vorliegenden Erfindung bestehen die erfindungsgemäßen Zusammensetzungen ausschließlich aus den in dieser Anmeldung explizit genannten essentiellen und optionalen Komponenten.

Im Rahmen der vorliegenden Erfindung enthalten die Zusammensetzungen weniger als 70 µl⁻¹, vorzugsweise weniger als 50 µl⁻¹, bevorzugt weniger als 25 µl⁻¹, günstigerweise weniger als 10 µl⁻¹, besonders bevorzugt weniger als 5,0 µl⁻¹, ganz besonders bevorzugt weniger als 1,0 µl⁻¹, insbesondere keine, Amnionzellen.

Weiterhin enthalten die erfindungsgemäßen Zusammensetzungen vorzugsweise weniger als 750 µl⁻¹, vorteilhafterweise weniger als 500 µl⁻¹, bevorzugt weniger als 250 µl⁻¹, günstigerweise weniger als 100 µl⁻¹, besonders bevorzugt weniger als 50,0 µl⁻¹, ganz besonders bevorzugt weniger als 25,0 µl⁻¹, noch mehr bevorzugt weniger als 10,0 µl⁻¹, zweckmäßigerweise weniger als 1,0 µl⁻¹, insbesondere keine, fetale Zellen.

Darüber hinaus enthalten die erfindungsgemäßen Zusammensetzungen vorzugsweise weniger als 70 µl⁻¹, vorteilhafterweise weniger als 50 µl⁻¹, bevorzugt weniger als 25 µl⁻¹, günstigerweise weniger als 10 µl⁻¹, besonders bevorzugt weniger als 5,0 µl⁻¹, ganz besonders bevorzugt weniger als 1,0 µl⁻¹, insbesondere keine, Trophoplastenzellen.

Ferner enthalten die erfindungsgemäßen Zusammensetzungen vorzugsweise weniger als 70 µl⁻¹, vorteilhafterweise weniger als 50 µl⁻¹, bevorzugt weniger als 25 µl⁻¹, günstigerweise weniger als 10 µl⁻¹, besonders bevorzugt weniger als 5,0 µl⁻¹, ganz besonders bevorzugt weniger als 1,0 µl⁻¹, insbesondere keine, lebenden Zellen.

Weiterhin enthalten die erfindungsgemäßen Zusammensetzungen vorzugsweise weniger als 70 µl⁻¹, vorteilhafterweise weniger als 50 µl⁻¹, bevorzugt weniger als 25 µl⁻¹, günstigerweise weniger als 10 µl⁻¹, besonders bevorzugt weniger als 5,0 µl⁻¹, ganz besonders bevorzugt weniger als 1,0 µl⁻¹, insbesondere keine, Stammzellen.

Zusätzlich enthalten die erfindungsgemäße Zusammensetzungen vorzugsweise weniger als 100 ngl⁻¹, vorteilhafterweise weniger als 50 ngl⁻¹, bevorzugt weniger als 1 ngl⁻¹, günstigerweise weniger als 500 pgl⁻¹, besonders bevorzugt weniger als 100 pgl⁻¹, ganz besonders bevorzugt weniger als 30 pgl⁻¹, insbesondere keine, Zell-freie, fetale DNA.

Im Rahmen einer ganz besonders bevorzugten Variante der vorliegenden Erfindung umfassen die Zusammensetzungen keine menschlichen und vorzugsweise auch keine tierischen Gewebereste, insbesondere keine infektiösen Bestandteile und keine menschliche oder tierische DNA. Besonders bevorzugt enthalten sie auch keine infektiösen tierischen Bestandteile und keine tierische DNA.

Weiterhin sind die erfindungsgemäßen Zusammensetzungen vorzugsweise steril und umfassen vorzugsweise keine vermehrungsfähigen Keime, insbesondere keine Mikroorganismen oder Viren. Dies kann durch die üblichen Sterilisationsverfahren erreicht bzw. sichergestellt werden.

Darüber hinaus sind die erfindungsgemäßen Zusammensetzungen vorzugsweise pyrogenfrei. Als *"pyrogen"* werden jene Stoffe bezeichnet, die entzündlich wirken. Hierzu gehören insbesondere die Stoffe, die bei parenteraler Gabe Fieber erzeugen können, insbesondere Endotoxine gramnegativer Bakterien, Bestandteile grampositiver Bakterien, Viruspyrogene, Pyrogene als Bestandteile von Pilzen, Pyrogene nicht biologischen Ursprungs.

Die Herstellung der erfindungsgemäßen Zusammensetzungen kann auf an sich bekannte Weise durch Mischen der Komponenten in den jeweiligen Einsatzmengen erfolgen. Alternativ ist auch das Auflösen einer Zusammensetzung, die die gewünschten Komponenten in den gewünschten Mengenverhältnissen enthält, in Wasser oder die Verdünnung eines wässrigen Konzentrates auf die gewünschte Konzentration denkbar.

Ansonsten sollte auf eine möglichst sterile Herstellung der wässrigen Zusammensetzungen geachtet werden, damit die resultierenden Zusammensetzungen auch nach längerer Lagerung, von beispielsweise einem Jahr oder länger, möglichst frei von vermehrungsfähigen Keimen, insbesondere von Mikroorganismen und Viren, sind.

Die Verabreichung der erfindungsgemäßen wässrigen Zusammensetzungen kann einmalig, periodisch oder auch kontinuierlich erfolgen, wobei die Vorteile der vorliegenden Erfindung insbesondere bei einer kontinuierlichen Verabreichung zu beobachten sind.

Ansonsten kann die Verabreichung auf an sich bekannte Weise durchgeführt werden. Um Wiederholungen zu vermeiden, wird in diesem Zusammenhang auf die bekannten Amnioninfusionsverfahren (mit isotonischer Salzlösung, insbesondere mit physiologischer Kochsalzlösung), insbesondere auf die einleitend genannten Veröffentlichungen, verwiesen.

Für die kontinuierliche Amnioninfusion ist die Verwendung eines Portsystems, insbesondere eines subkutan implantierten Fruchtwasserersatzportsystems, empfehlenswert. Entsprechende Port-Systeme werden in der Literatur beschrieben (Tchirikov M, Steetskamp J, Hohmann M, Koelbl H. Long-term amnioinfusion through a subcutaneously implanted amniotic fluid replacement port system for treatment of PPROM in humans. Eur J Obstet Gynecol Reprod Biol. 2010; 152:30-33 und in WO2011154128), deren Offenbarung hierin durch Bezugnahme explizit mit aufgenommen wird.

Die Infusionsgeschwindigkeit ist bei kontinuierlicher Amnioninfusion vorzugsweise größer 1,0 ml/h, bevorzugt größer 10,0 ml/h, besonders bevorzugt größer 50,0 ml/h, insbesondere größer 75,0 ml/h. Weiterhin ist sie vorzugsweise kleiner 1000,0 ml/h, bevorzugt kleiner 500,0 ml/h, besonders bevorzugt kleiner 250 ml/h, insbesondere kleiner 125,0 ml/h.

Die Dauer der kontinuierlichen Amnioninfusion unterliegt grundsätzlich keinen besonderen Beschränkungen. Allerdings kommen die Vorteile der vorliegenden Erfindung insbesondere bei Lanzzeitverabreichung der erfindungsgemäßen Zusammensetzungen zum tragen. Besonders vorteilhaft ist die erfindungsgemäße Vorgehensweise für kontinuierliche Amnioninfusionen mit einer Dauer von mindestens 30 min, bevorzugt mindestens 1h, zweckmäßigerweise mindestens 2h, günstigerweise mindestens 4h, besonders bevorzugt mindestens 12h, noch mehr bevorzugt mindestens 24h, vorteilhafterweise mindestens 3 Tage, vorzugsweise mindestens eine Woche, ganz besonders bevorzugt mindestens 3 Wochen, insbesondere mindestens 6 Wochen. Dabei sind gelegentliche Unterbrechungen der kontinuierlichen Verabreichung möglich. Diese sollten aber zweckmäßigerweise nicht länger als 100% der Zeit, bevorzugt nicht länger als 50% der Zeit, seit der letzten Unterbrechung dauern.

Für den Fall, dass die erfindungsgemäße Zusammensetzungen nicht vollkommen homogen sind und beispielsweise als wässrige Suspensionen oder Emulsionen vorliegen, werden sie möglichst frisch, d. h. möglichst direkt vor ihrer Anwendung hergestellt. Dementsprechend werden Zusammenstellungen, umfassend die Einzelkomponenten der erfindungsgemäßen wässrigen Zusammensetzungen in mindestens zwei unterschiedlichen Fraktionen, im Rahmen der vorliegenden Erfindung ebenfalls unter Schutz gestellt.

Im Rahmen einer besonders bevorzugten Variante der vorliegenden Erfindung werden die Konzentrationen der Bestandteile der Zusammensetzungen während ihrer vorzugsweise kontinuierlichen Verabreichung mit der Zeit geändert, um dem jeweiligen Alter des Fötus Rechnung zu tragen. So kann die Surfactant-Konzentration, insbesondere die Phospholipid-Konzentration ggf. erhöht werden, um die Lungenreifung zu beschleunigen. Weiterhin kann die Konzentration von einem oder mehreren Surfactant-Proteinen erhöht werden, um die Lungenreifung zu fördern.

Die Zusammensetzung gemäß dem ersten Aspekt der vorliegenden Erfindung kann nicht nur bei der Amnioninfusion vorteilhaft eingesetzt werden. Weitere Anwendungsgebiete dieser erfindungsgemäßen Zusammensetzung umfassen insbesondere alle medizinischen Anwendungen, bei denen eine vorzugsweise hypotone wässrige Zusammensetzung mit Phospholipiden eingesetzt wird. Besonders bevorzugte Anwendungsgebiete schließen das Spülen von Körperhöhlen, bevorzugt das Ausspülen von eitrigen Höhlen, insbesondere in der Lunge, wie z. B. im Rahmen einer bronchoalveolären Lavage, sowie das Spülen von mit Häuten, insbesondere mit Schleimhäuten, überzogenen Körperbereichen ein.

Im Folgenden wird die vorliegende Erfindung durch Beispiele und Vergleichsbeispiele weiter veranschaulicht, ohne dass hierdurch eine Beschränkung des Erfindungsgedankens erfolgen soll.

### Beispiele 1, 2, 3 und 4

In Tabelle 1 werden für die Zwecke der vorliegenden Erfindung besonders geeignete wässrige Zusammensetzungen für Amnioninfusionen, insbesondere für kontinuierliche Amnioninfusionen, als Beispiel angegeben.

**Tabelle 1: wässrige Zusammensetzungen für Amnioninfusionen**

| | Beispiel 1 | Beispiel 2 | Beispiel 3 | Beispiel 4 |
|---|---|---|---|---|
| Osmolarität [mosm/l] | 240-300 | 271 | 271 | 271 |
| Natrium [mmol/l] | 130-145 | 134,8 | 134,8 | 134,8 |
| Kalium [mmol/l] | 3,5-4,5 | 3,9 | 3,9 | 3,9 |
| Calcium [mmol/l] | 1-2,5 | 1,9 | 1,9 | 1,9 |
| Magnesium [mmol/l] | 0-2 | 0,57 | 0,57 | 0,57 |
| Chlorid [mmol/l] | 100-120 | 109,5 | 109,5 | 109,5 |
| Harnstoff [mg/dl] | 0-10 | 10 | 10 | 10 |
| Harnsäure [mg/dl] | 0-5 | 3,5 | 3,5 | 3,5 |
| Phosphat [mg/dl] | 0-8 | 3,3 | 3,3 | 3,3 |
| Glucose [mg/dl] | 0-340 | <60,0 | <60,0 | <60,0 |
| Laktat [mmol/l] | 0-20 | 9,1 | 9,1 | 9,1 |
| Citrat [mg/l] | 0-100 | 66,5 | 66,5 | 66,5 |
| Hydrogencarbonat [mmol/l] | 5-100 | 16,9 | 16,9 | 16,9 |
| Gesamt-Eiweiß [g/l] | 0-10 | 3 | 3 | 3 |
| Albumin [g/dl] | 0-5 | <2 | <2 | <2 |
| Kupfer [µg/dl] | 0-150 | 16 | 16 | 16 |
| Selen [µg/dl] | 0-50 | <13,3 | <13,3 | <13,3 |
| Zink [µg/dl] | 0-30 | 10-24 | 10-24 | 10-24 |
| SURFACTANT Fraktionen | | | | |
| Lipide insgesamt [mg/l] | 0-2000 | 100-339 | 100-339 | 500-1695 |
| Phosphatidylcholin (PC) [mg/l] | 0-1000 | 93 | 93 | 465 |
| (bevorzugt 80% der Lipide insgesamt) | | | | |
| Dipalmitoylphosphatidylcholin [mg/l] | 0-600 | 47 | 47 | 235 |
| (bevorzugt 36%-60% von PC) | | | | |
| Palmitoylmyristolphosphatidylcholin [mg/l] | 0-200 | 6,2 | 6,2 | 31 |
| Phosphatidylglyzerin [mg/l] | 0-200 | 11 | 11 | 55 |
| (bevorzugt 12% von PC) | | | | |
| Phosphatidylethanolamin [mg/l] | 0-100 | 4,5 | 4,5 | 22,5 |
| (bevorzugt 5% von PC) | | | | |
| Phosphatidylinositol [mg/l] | 0-70 | 4 | 4 | 20 |
| (bevorzugt 4% von PC) | | | | |
| Phosphatidylserin [mg/l] (bevorzugt 1,5% von PC) | 0-50 | 1,4 | 1,4 | 7 |
| Sphingomyelin [mg/l] | 0-1000 | 1 | 10-40 | 50-100 |
| (bevorzugt 1% von PC) | | | | |
| Lysophospholipid [mg/l] | 0-10 | 0,5 | 0,5 | 2,5 |
| (bevorzugt <1% von PC) | | | | |
| L/S-Verhältnis | > 1,5 | > 2,0 | > 2,0 | > 2,0 |
| Cholesterol [mg/l] | 0-100 | 5 | 5 | 25 |
| (bevorzugt 5%-10% der Lipide insgesamt) | | | | |
| Surfactant Protein A [mg/l] | 0-500 | 5,6 | 5,6 | 28 |
| Surfactant Protein B [mg/l] | 0-100 | 0,54 | 0,54 | 2,7 |
| Surfactant Protein D [µg/l] | 0-1000 | 11,8 | 11,8 | 59 |
| Apoprotein B g/l | 0-1 | 0,12 | 0,12 | 0,6 |
| Lipoprotein mg/l | 0-1000 | 84,53 | 84,53 | 422 |
| Wasser | Rest | Rest | Rest | Rest |
| pH | 7-8,6 | 8,35 | 8,35 | 8,35 |
| Amnionzellen [µl] | 0,0 | 0,0 | 0,0 | 0,0 |
| fetale Zellen [µl] | 0,0 | 0,0 | 0,0 | 0,0 |
| Trophoplastenzellen [µl] | 0,0 | 0,0 | 0,0 | 0,0 |
| lebende Zellen [µl] | 0,0 | 0,0 | 0,0 | 0,0 |
| Stammzellen [µl] | 0,0 | 0,0 | 0,0 | 0,0 |
| fetale DNA [pgl⁻¹] | 0,0 | 0,0 | 0,0 | 0,0 |
| Nicht-fetale DNA [pgl⁻¹] | <30 | <30 | <30 | <30 |

Die Glucosekonzentration in den erfindungsgemäßen Beispielen liegt vorzugsweise im Bereich von 0,0 mg/dl bis 340,0 mg/dl. Sie ist bevorzugt kleiner 300,0 mg/dl, günstigerweise kleiner 200,0 mg/dl, zweckmäßigerweise kleiner 100,0 mg/dl, insbesondere kleiner 60,0 mg/dl. Im Rahmen einer ganz besonders bevorzugten Ausführungsform der vorliegenden Erfindung enthalten die Beispiele weniger als 50,0 mg/dl, günstigerweise weniger als 25,0 mg/dl, zweckmäßigerweise weniger als 10,0 mg/dl, insbesondere keine, Glucose.

### Vergleichsbeispiel 1

Nach einer kontinuierlichen Amnioninfusion von 2000 ml physiologischer Kochsalzlösung/24h über ein Port-System beim PPROM kam es zu einem Zwischenfall; die gesunde Patientin entwickelte einen Hypertonus, den man mit einer starken i. v. Medikation akut behandelt musste. Es wird davon ausgegangen, dass dieses Phänomen durch einen Anstieg von Angiotensin II durch die Aktivierung des RAAS-Systems des Feten durch das enthaltene Salz der NaCI-Lösung verursacht wurde. Die Mutter wurde mit den vasoaktiven Substanzen des Feten durch die Placenta ungünstig beeinflusst.

### Vergleichsbeispiel 2

Bei einer kontinuierlichen Sterofundin-Amnioninfusion von 2000 ml/24h über ein Port-System kam es zu einer deutlich erhöhten Diurese der Mutter. Der Fetus trinkt die Flüssigkeit, die durch die Plazenta zur Mutter weitergeleitet und über die Nieren der Frau ausgeschieden wird. Dadurch ist der Effekt der Lavage bei PPROM bei der intrauterinen Gabe der Sterofundin-Lösung signifikant reduziert.

### Beispiel 5

Eine kontinuierliche Amnioninfusion von 2000 ml Beispiel 4/24 verlief ohne Probleme. Die Lösung wurde sehr gut vertragen und es gab keine Komplikationen. Die Schwangerschaft konnte auf diese Weise deutlich verlängert und so das Risiko einer Frühgeburt deutlich verringert werden. Eine Schädigung von Organen des Fötus, vor allem seiner Haut, seiner Augen und seiner Verdauungsorgane, insbesondere seiner Nieren, konnte nicht festgestellt werden.

## Patentansprüche

1. Wässrige Zusammensetzung zur Anwendung in einem Verfahren zur chirurgischen oder therapeutischen Behandlung des menschlichen oder tierischen Körpers oder in einem Diagnostizierverfahren, das am menschlichen oder tierischen Körper vorgenommen wird, wobei die Zusammensetzung osmotisch aktive Teilchen umfasst und 0,0 µl⁻¹ Amnionzellen enthält, wobei die Osmolarität der Zusammensetzung im Bereich von 240 mosm/l bis 300 mosm/l liegt, **dadurch gekennzeichnet, dass** die Zusammensetzungen aus den folgenden Komponenten besteht
▪ 130-145 mmol/l Natrium,
▪ 3,5-4,5 mmol/l Kalium,
▪ 1-2,5 mmol/l Calcium,
▪ 0-2 mmol/l Magnesium,
▪ 100-120 mmol/l Chlorid,
▪ 0-10 mg/dl Harnstoff,
▪ 0-5 mg/dl Harnsäure,
▪ 0-8 mg/dl Phosphat,
▪ 0-340 mg/dl Glucose,
▪ 0-20 mmol/l Laktat,
▪ 0-100 mg/l Citrat,
▪ 5-100 mmol/l Hydrogencarbonat,
▪ 0-10 g/l Gesamt-Eiweiß,
▪ 0-5 g/dl Albumin,
▪ 0-150 µg/dl Kupfer,
▪ 0-50 µg/dl Selen,
▪ 0-30 µg/dl Zink,
▪ 0-2000 mg/l Lipide insgesamt,
▪ 0-1000 mg/l Phosphatidylcholin (PC),
▪ 0-600 mg/l Dipalmitoylphosphatidylcholin,
▪ 0-200 mg/l Palmitoylmyristolphosphatidylcholin,
▪ 0-200 mg/l Phosphatidylglyzerin,
▪ 0-100 mg/l Phosphatidylethanolamin,
▪ 0-70 mg/l Phosphatidylinositol,
▪ 0-50 mg/l Phosphatidylserin,
▪ 0-1000 mg/l Sphingomyelin,
▪ 0-10 mg/l Lysophospholipid,
▪ 0-100 mg/l Cholesterol,
▪ 0-500 mg/l Surfactant Protein A,
▪ 0-100 mg/l Surfactant Protein B,
▪ 0-1000 µg/l Surfactant Protein D,
▪ 0-1 g/l Apoprotein B,
▪ 0-1000 mg/l Lipoprotein,
▪ <30 pgl⁻¹ nicht-fetale DNA,
▪ Wasser Rest
wobei das L/S-Verhältnis > 1,5 ist, sofern die Zusammensetzung beide Komponenten enthält, und der pH 7-8,6 ist.

2. Wässrige Zusammensetzung zur Anwendung in einem Verfahren nach Anspruch 1, **dadurch gekennzeichnet, dass** die Zusammensetzung mindestens ein Phospholipid enthält.

3. Wässrige Zusammensetzung zur Anwendung in einem Verfahren nach Anspruch 1 oder 2, **dadurch gekennzeichnet, dass** die Zusammensetzung mindestens ein Surfactant-Protein enthält.

4. Zusammensetzung zur Anwendung in einem Verfahren nach mindestens einem der vorangehenden Ansprüche zur Anwendung bei einer Amnioninfusion.

5. Zusammensetzung zur Anwendung in einem Verfahren nach mindestens einem der vorangehenden Ansprüche zur Behandlung eines vorzeitigen Blasensprungs.

6. Zusammensetzung zur Anwendung in einem Verfahren nach Anspruch 5 zur Anwendung bei einer kontinuierlichen Amnioninfusion.

7. Zusammensetzung zur Anwendung in einem Verfahren nach mindestens einem der vorangehenden Ansprüche, **dadurch gekennzeichnet, dass** die Zusammensetzung mindestens ein Phosphatidylcholin enthält.

8. Zusammensetzung zur Anwendung in einem Verfahren nach Anspruch 7, **dadurch gekennzeichnet, dass** der Gewichtsanteil von Phosphatidylcholin, bezogen auf alle Lipide, größer 50,0 Gew.-% ist.

9. Zusammensetzung zur Anwendung in einem Verfahren nach mindestens einem der vorangehenden Ansprüche, **dadurch gekennzeichnet, dass** die Zusammensetzung Sphingomyelin enthält.

10. Zusammensetzung zur Anwendung in einem Verfahren nach Anspruch 9, **dadurch gekennzeichnet, dass** das Gewichtsverhältnis von Phospholipiden zu Sphingomyelin größer 1,5 ist.

11. Zusammensetzung zur Anwendung in einem Verfahren nach Anspruch 3, **dadurch gekennzeichnet, dass** der Gewichtsanteil des Surfactant-Proteins, bezogen auf das Gesamtgewicht der Zusammensetzung, größer 1,0 mg/l ist.

12. Zusammensetzung zur Anwendung in einem Verfahren nach Anspruch 2, **dadurch gekennzeichnet, dass** der Gewichtsanteil aller Phospholipide, bezogen auf das Gesamtgewicht der Zusammensetzung, größer 120,0 mg/l ist.

13. Zusammenstellung zur Anwendung in einem Verfahren zur chirurgischen oder therapeutischen Behandlung des menschlichen oder tierischen Körpers oder in einem Diagnostizierverfahren, das am menschlichen oder tierischen Körper vorgenommen wird, umfassend die Einzelkomponenten einer Zusammensetzung nach mindestens einem der vorangehenden Ansprüche in mindestens zwei unterschiedlichen Fraktionen.

14. Wässrige Zusammensetzung zur Anwendung bei einer kontinuierlichen Amnioninfusion zur Behandlung eines vorzeitigen Blasensprungs, wobei die Zusammensetzung osmotisch aktive Teilchen umfasst und weniger als 70 µl⁻¹ Amnionzellen enthält, **dadurch gekennzeichnet, dass** die Osmolarität der Zusammensetzung im Bereich von 240,0 mosm/l bis kleiner 308,0 mosm/l liegt und die Chloridionenkonzentration in der Zusammensetzung höchstens 130,0 mmol/l ist.

## Claims

1. An aqueous composition for application in a method for the surgical or therapeutic treatment of the human or animal body or in a diagnostic method performed on the human or animal body, wherein the composition comprises osmotically active particles and contains 0.0 amniotic cells per µl wherein the osmolarity of the composition is in the range from 240 mosm/l to 300 mosm/l, **characterized in that** the composition consists of the following components
▪ 130-145 mmol/l of sodium,
▪ 3.5-4.5 mmol/l of potassium,
▪ 1-2.5 mmol/l of calcium,
▪ 0-2 mmol/l of magnesium,
▪ 100-120 mmol/l of chloride,
▪ 0-10 mg/dl of urea,
▪ 0-5 mg/dl of uric acid,
▪ 0-8 mg/dl of phosphate,
▪ 0-340 mg/dl of glucose,
▪ 0-20 mmol/l of lactate,
▪ 0-100 mg/l of citrate,
▪ 5-100 mmol/l of hydrogencarbonate,
▪ 0-10 g/l of total protein,
▪ 0-5 g/dl of albumin,
▪ 0-150 µg/dl of copper,
▪ 0-50 µg/dl of selenium,
▪ 0-30 µg/dl of zinc,
▪ 0-2000 mg/l of total lipids,
▪ 0-1000 mg/l of phosphatidylcholine (PC),
▪ 0-600 mg/l of dipalmitoylphosphatidylcholine,
▪ 0-200 mg/l of palmitoylmyristolphosphatidylcholine,
▪ 0-200 mg/l of phosphatidylglycerol,
▪ 0-100 mg/l of phosphatidylethanolamine,
▪ 0-70 mg/l of phosphatidylinositol,
▪ 0-50 mg/l of phosphatidylserine,
▪ 0-1000 mg/l of sphingomyelin,
▪ 0-10 mg/l of lysophospholipid,
▪ 0-100 mg/l of cholesterol,
▪ 0-500 mg/l of surfactant protein A,
▪ 0-100 mg/l of surfactant protein B,
▪ 0-1000 µg/l of surfactant protein D,
▪ 0-1 g/l of apoprotein B,
▪ 0-1000 mg/l of lipoprotein,
▪ <30 pgl⁻¹ of nonfetal DNA,
▪ the rest being water,
wherein the L/S ratio is > 1.5, provided that the composition contains both components, and the pH is 7-8.6.

2. The aqueous composition for application in a method as claimed in claim 1, **characterized in that** the composition contains at least one phospholipid.

3. The aqueous composition for application in a method as claimed in claim 1 or 2, **characterized in that** the composition contains at least one surfactant protein.

4. The composition for application in a method as claimed in at least one of the preceding claims for application in amnioinfusion.

5. The composition for application in a method as claimed in at least one of the preceding claims for the treatment of preterm premature rupture of membranes.

6. The composition for application in a method as claimed in claim 5 for application in continuous amnioinfusion.

7. The composition for application in a method as claimed in at least one of the preceding claims, **characterized in that** the composition contains at least one phosphatidylcholine.

8. The composition for application in a method as claimed in claim 7, **characterized in that** the weight fraction of phosphatidylcholine, based on all lipids, is greater than 50.0% by weight.

9. The composition for application in a method as claimed in at least one of the preceding claims, **characterized in that** the composition contains sphingomyelin.

10. The composition for application in a method as claimed in claim 9, **characterized in that** the weight ratio of phospholipids to sphingomyelin is greater than 1.5.

11. The composition for application in a method as claimed in claim 3, **characterized in that** the weight fraction of the surfactant protein, based on the total weight of the composition, is greater than 1.0 mg/l.

12. The composition for application in a method as claimed in claim 2, **characterized in that** the weight fraction of all phospholipids, based on the total weight of the composition, is greater than 120.0 mg/l.

13. A combination for application in a method for the surgical or therapeutic treatment of the human or animal body or in a diagnostic method performed on the human or animal body, comprising the individual components of a composition as claimed in at least one of the preceding claims in at least two different fractions.

14. An aqueous composition for application in continuous amnioinfusion for the treatment of preterm premature rupture of membranes, wherein the composition comprises osmotically active particles and contains fewer than 70 amniotic cells per µl, **characterized in that** the osmolarity of the composition is in the range from 240.0 mosm/l to less than 308.0 mosm/l and the chloride ion concentration in the composition is not more than 130.0 mmol/l.

## Revendications

1. Composition aqueuse destinée à être utilisée dans un procédé pour le traitement chirurgical ou thérapeutique du corps humain ou animal ou dans un procédé de diagnostic qui est mis en oeuvre sur le corps humain ou animal, où la composition comprend des particules actives du point de vue osmotique et contient 0,0 µl⁻¹ de cellules amniotiques, où l'osmolarité de la composition est située dans le domaine de 240 mosm/l à 300 mosm/l, **caractérisée en ce que** la composition consiste en les composants suivants
▪ 130 - 145 mmol/l de sodium,
▪ 3,5 - 4,5 mmol/l de potassium,
▪ 1 - 2,5 mmol/l de calcium,
▪ 0 - 2 mmol/l de magnésium,
▪ 100 - 200 mmol/l de chlorure,
▪ 0 - 10 mg/dl d'urée,
▪ 0 - 5 mg/dl d'acide urique,
▪ 0 - 8 mg/dl de phosphate,
▪ 0 - 340 mg/dl de glucose,
▪ 0 - 20 mmol/l de lactate,
▪ 0 - 100 mg/l de citrate,
▪ 5 - 100 mmol/l d'hydrogénocarbonate,
▪ 0 - 10 g/l de protéines totales,
▪ 0 - 5 g/dl d'albumine,
▪ 0 - 150 µg/dl de cuivre,
▪ 0 - 50 µg/dl de sélénium,
▪ 0 - 30 µg/dl de zinc,
▪ 0 - 2000 mg/l de lipides totaux,
▪ 0 - 1000 mg/l de phosphatidylcholine (PC),
▪ 0 - 600 mg/l de dipalmitoylphosphatidylcholine,
▪ 0 - 200 mg/l de palmitoylmyristolphosphatidylcholine,
▪ 0 - 200 mg/l phosphatidylglycérine,
▪ 0 - 100 mg/l de phosphatidyléthanolamine,
▪ 0 - 70 mg/l de phosphatidylinositol,
▪ 0 - 50 mg/l de phosphatidylsérine,
▪ 0 - 1000 mg/l de sphingomyéline,
▪ 0 - 10 mg/l de lysophospholipide,
▪ 0 - 100 mg/l de cholestérol,
▪ 0 - 500 mg/l de protéine tensioactive A,
▪ 0 - 100 mg/l de protéine tensioactive B,
▪ 0 - 1000 µg/l de protéine tensioactive D,
▪ 0 - 1 g/l d'apoprotéine B,
▪ 0 - 1000 mg/l de lipoprotéine,
▪ < 30 pgl⁻¹ d'ADN non foetal,
▪ reste d'eau
où le rapport L/S est > 1,5, à condition que la composition contienne les deux composants, et le pH est 7-8,6.

2. Composition aqueuse destinée à être utilisée dans un procédé selon la revendication 1, **caractérisée en ce que** la composition contient au moins un phospholipide.

3. Composition aqueuse destinée à être utilisée dans un procédé selon la revendication 1 ou 2, **caractérisée en ce que** la composition contient au moins une protéine tensioactive.

4. Composition destinée à être utilisée dans un procédé selon au moins l'une des revendications précédentes destinée à être utilisée lors d'une amnio-infusion.

5. Composition destinée à être utilisée dans un procédé selon au moins l'une des revendications précédentes pour le traitement d'une rupture prématurée des membranes.

6. Composition destinée à être utilisée dans un procédé selon la revendication 5 destinée à être utilisée lors d'une amnio-infusion continue.

7. Composition destinée à être utilisée dans un procédé selon au moins l'une des revendications précédentes, **caractérisée en ce que** la composition contient au moins une phosphatidylcholine.

8. Composition destinée à être utilisée dans un procédé selon la revendication 7, **caractérisée en ce que** la fraction en poids de phosphatidylcholine, par rapport à tous les lipides, est supérieure à 50,0 % en poids.

9. Composition destinée à être utilisée dans un procédé selon au moins l'une des revendications précédentes, **caractérisée en ce que** la composition contient de la sphingomyéline.

10. Composition destinée à être utilisée dans un procédé selon la revendication 9, **caractérisée en ce que** la proportion en poids de phospholipides par rapport à la sphingomyéline est supérieure à 1,5.

11. Composition destinée à être utilisée dans un procédé selon la revendication 3, **caractérisée en ce que** la fraction en poids de protéine tensioactive, par rapport au poids total de la composition, est supérieure à 1,0 mg/l.

12. Composition destinée à être utilisée dans un procédé selon la revendication 2, **caractérisée en ce que** la fraction en poids de tous les phospholipides, par rapport au poids total de la composition, est supérieure à 120,0 mg/l.

13. Composition destinée à être utilisée dans un procédé pour le traitement chirurgical ou thérapeutique du corps humain ou animal ou dans un procédé de diagnostic qui est mis en oeuvre sur le corps humain ou animal, comprenant les composants individuels d'une composition selon au moins l'une des revendications précédentes dans au moins deux fractions différentes.

14. Composition aqueuse destinée à être utilisée lors d'une amnio-infusion continue pour le traitement d'une rupture prématurée des membranes, où la composition comprend des particules actives du point de vue osmotique et contient moins de 70 µl⁻¹ de cellules amniotiques, **caractérisée en ce que** l'osmolarité de la composition est située dans le domaine de 240,0 mosm/l à moins de 308,0 mosm/l et la concentration d'ions chlorure dans la composition est d'au plus 130,0 mmol/l.
